# EUROPEAN PATENT SPECIFICATION

(11) **EP 2 733 197 B1**
(45) Date of publication and mention of the grant of the patent: **26.08.2015**
(21) Application number: 12461557.6
(22) Date of filing: 19.11.2012
(51) Int. Cl.: C12M 1/107

(54) **Biogas generating plant with tunnel fermentation chamber and installations to produce and utilise biogas**
Biogaserzeugungsanlage mit Tunnelgärungskammer und Installationen zur Herstellung und Verwendung von Biogas
Installation de production de biogaz à chambre de fermentation en tunnel et installations pour produire et utiliser du biogaz

(43) Date of publication of application: 21.05.2014
(73) Proprietor: Fotyga, Ryszard Aleksander, 80-255 Gdansk (PL)
(72) Inventor: Fotyga, Ryszard Aleksander, 80-255 Gdansk (PL)
(74) Representative: Sielewiesiuk, Jakub

(56) References cited:
- WO-A1-2008/150178
- DE-A1- 19 947 339
- DE-U1- 20 319 847
- FR-A- 2 470 159
- US-A- 4 436 818

## Description

The object of the invention is a biogas generating plant with tunnel fermentation chamber and installations to produce and utilise biogas, designated for application in agriculture, particularly on small farms for the production of biogas in the methane biomass fermentation process, with the possibility of periodic use of the biogas plant construction for the cultivation of plants.

From the study titled: "Methane fermentation, technology, equipment, and examples", M. Poczatek, M. Janik (source: www.en4.pl) the technology introduced by the Bekon company for the production of biogas from biomass based on the process of percolation with the application of continuous washing out of the charge in the reactor is known. The percolation reactor is a reinforced concrete prism with a large, gas-tight gate for loading. The mixing function in this reactor is performed by the circulation of process liquids, which are sprayed on the waste. This liquid enables the propagation of bacterial flora and dissolves organic compounds accelerating their breakdown. The system is equipped with two sources of heat: exchange in liquid container and also an incorporated installations in box construction. The intensity of irrigation is controlled by microprocessor. The charge loading solution is the use of a 'Tur' type loader. The charge is a single load of biomass without preliminary processing. The productivity of the process depends on the quality of the charge. Solution process enables the obtaining of biogas with methane value of up to 80%. Energy consumption used in the heating of the charge amounts to approximately 10%.

The DE 202005005077 U1 fermentation container, is a known fermentation container for the production of biogas from organic wastes. The container shape may be square, rectangular or oval or round. The container has a bottom sloped at a slight angle in the direction of a channel draining excess fluid. Fermentation of biomass in the container takes place in the presence of bacterial flora (micro organism cultures) under a thermo-insulation covering, above which is placed a gas impermeable screen/cover. The space between the thermo-insulation covering and the cover is used as a biogas store. The cover, under which the biogas is collected, is sealed on the edge of the container with the aid of a siphon closure. The fermentation process can be controlled by the regulation of the amount of liquid introduced to the inside, which is then extracted from the container. The installations composition may include a liquid recirculation system, heat exchangers, pumps, ventilators, hot air biomass heating system and cogeneration system. The possibility is also foreseen of compressing and storing compressed biogas and also the possibility of connecting other renewable energy systems to the cogeneration system.

The DE 20319847 U1 is known as a biogas generating plant with increased dimensions. The biogas generating plant has a tunnel construction with a gas tight base and vertical sides, on which is supported the roof construction that is equipped with two layers of elastic gas proof material or foil. The space between the layers of foil may be used at as a thermo-insulation barrier or as a temporary gas store. In this solution the roof construction is permanently secured to the sidewalls and loading takes place after opening the sealed flap from the upper side.

The equipment with the Polish patent no. 166913 is known as equipment for the production of biogas from solid agricultural waste, having a fermentation chamber with thermal insulation cover secured on an incline to the foundation with the aid of a hinge element. The edges of the cover are extended by sealing panels, which are submerged in the liquid supplementing the container sealing executed in the foundation. The equipment is supplied with a heater and the foundation has thermal insulation.

The purpose of the invention is the development of a biogas construction for the processing of biomass available in various forms on farms, comprising dry and pulp biomass, biomass pulp occurs as a by-product of animal production, with the possibility of use particularly in the conversion of post-fermentation deposits into compost for plant production, and also development and installations enabling the controlled course of methane fermentation in biogas plant and utilisation of biogas for the needs of the farm in a cogeneration system.

The biogas generation plant with tunnel fermentation chamber, is equipped with a biomass container with a thermally insulated base and essentially vertical foundation walls, installations for moistening biomass with a process liquid, installations for the recirculation of seepage water, arch roof covering secured to load bearing construction and supported on essentially vertical foundation walls of biomass container, gas proof gable walls, and biogas store created by the double layer foil roof covering with inlet to the gas store placed on the upper part of the fermentation chamber, according to the invention it is typified by the two layers of foil, upper and lower, creating biogas storage capacity, being secured to opposite sides of rigid arch roof cover construction, the lower foil at a height of approximately 1/4 of the height of the arch load-bearing construction has gas slots, and the inlet channel is connected to a duct fan forcing circulation of biogas to the biogas store, wherein the lower edges of the roof covering are supported on the upper part of the essentially vertical foundation walls of the biomass container, while the installations for moistening biomass with process liquid have moisturising nozzles, placed in the biomass loading zone at variable heights, depending on the height of the biomass loading zone, preferably in the intermediate zone, furthermore in the biomass loading zone is a channel for pulp, preferably created by a perforated barrier supported on the base of the biomass container or constituting a perforated pipe supported or resting freely in the biomass layer.

The pulp channel may have various shapes and may be laid above the biomass container base.

In appropriate biogas generating plant construction, the lower edges of the roof cover are slideably mounted and create a gas proof connection with the appropriately shaped upper part of the vertical foundation walls, particularly with the aid of slides or guide rails comprising channel sections

Alternatively and appropriately the lower edges of the roof covering are secured with slides in recesses executed in the upper parts of the vertical foundation walls. These recesses form a gas tight connection with the lower edges, especially with siphon connections.

A heating conduit should preferably be placed in the lowest point of the biogas store.

Preferably the moisturising nozzles are placed in the biomass-loading zone at various levels between the biomass container and the upper part of the fermentation chamber. Alternatively preferably moisturising nozzles are placed at one level in the zone between the biomass container and the upper part of the fermentation chamber.

In the situation when the pulp channel is separated by a perforated barrier supported by the biomass container base it is preferably placed between the biomass container side zones and the perforated barrier has the shape of a reversed letter U.

Preferably in the upper part of the pulp channel an internal pipeline is placed conveying the pulp, and in the lower part of the pulp channel is placed an auger conveyor to take compacted pulp to the exterior.

Preferably in one of the examples of the invention utilisation, the biomass container has a rectangular shape when viewed from above, while the pulp channel is placed lengthwise in the central part of the container, in parallel with its longer sides.

Preferably the biomass container is equipped with aeration installations with perforated nozzles mounted in the side zones by the biomass container base, supplied by aeration pipeline.

Preferably the biogas installations according to the invention possess a coil placed in the biomass layer, to remove surplus heat from the fermentation chamber or for heating the bed.

Preferably the stiff bowed support construction of the roof covering be placed in a gas-tight foil sleeve, insulating the construction from the gaseous atmosphere in the biogas store.

In such a case, preferably the foil sleeve referred to should have inspection openings, in order to serve as inlets for appropriate gases protecting the construction against damage and to control the moisture in the atmosphere inside the sleeve.

Preferably the foundation walls are made of materials limiting the escape of heat from inside the biogas plant, e.g. from thermoinsulating material, walled or made from thermoinsulating panels placed between support pillars.

The installations for the production and utilisation of biogas, comprising tunnel fermentation chamber, constitute the internal space of the biogas installations with a convex roof cover spread across the arched support structure, the biogas store formed by two layers of roof covering foil with the inlet to the biogas store situated in the upper part of the fermentation chamber, installations for moisturising biomass with a process liquid , seepage water recirculation system, cogeneration system powered by biogas brought by gas pipeline from gas store and an automation system to control process of production and utilisation of biogas, according to the invention it is typified by two layers of foil, upper and lower, forming biogas store, they are secured on opposite sides of stiff arched roof covering support construction, the lower foil has gas slots at approximately 1/4 of the height of the arch construction, and there is a ventilator connected to the inlet channel for single circulation of biogas to the biogas store, while the biomass moisturising installations have a pipeline carrying biomass process liquid to moisturising nozzles placed in the biomass loading zone at varying heights, preferably in the intermediate zone and additionally in the biomass loading zone there is a channel for pulp, preferably created by a perforated barrier supported on the base of the biomass container or constituting a perforated suspended or supported pipe, or resting freely in the biomass layer, supplied by pulp from the internal pipeline situated in the pulp channel, wherein the seepage water recirculation installations include non-diaphragm heat and mass exchanger connecting by seepage water pipe from biomass container and by return pipe to process liquid biomass moisturising installations, wherein the non-diaphragm heat and mass exchanger have permanent mass of large heat capacity, through which seepage water is passed in the opposite direction to exhaust gases supplied to the permanent mass by an exhaust conduit from the cogeneration system

Preferably in the installations execution the seepage water pipe is connected with the perforated pipeline in the biomass container and seepage water pump connected on the compression side with sprinkler appliance over the permanent bed of non-diaphragm heat and mass exchanger, while below the permanent mass is placed a tank to receive water passing through mass, wherein the water from the tank is pumped into the return pipe by the return pump.

Preferably the internal pipeline is supplied by the first pump placed in the pulp container, especially in the manure collecting point, while the moisturising pipelines are supplied by the second water pump placed in the process liquid , especially in the upper layer of liquid in the manure collection point. Due to this solution liquid of a lesser permanent particle value is sucked from the upper layer in the manure collection point and shall not quickly muddy the substrates in the biomass-loading zone.

Preferably, the moisturising nozzles are situated in the biomass-loading zone at various levels between the base of the biomass container and the upper part of the fermentation chamber. Alternatively the moisturising nozzles are situated at one level in the intermediate zone between the biomass container and the upper part of the fermentation chamber.

In the case that the pulp channel is separated at the biomass container base by a perforated barrier, a channel preferably is situated between the side zones of the biomass container, and perforated barrier have the shape of a reversed letter U.

Preferably in the upper part of the pulp channel is placed the internal pipeline conveying the pulp and in the lower part of the channel is placed an auger conveyor to carry compacted post-fermentation deposits derived from pulp fermentation to the exterior.

Preferably, the biomass container is equipped with aeration installations with perforated nozzles mounted in side zones by the biomass container base, supplied by an aeration pipeline.

Preferably, a conduit connects the lower part of the biogas store with an external equalising carbon dioxide gas container, and additionally the equalising conduit is connected to a reversed ventilator forcing the through flow of gases.

The biogas plant construction, after the removal of the gable walls, enables moving the self-supporting roof construction in order to enable unhindered loading of biomass and removal of post-fermentation products. Appropriately shaped upper edges of the foundation walls serve as guide rails and seals. After completing the process of methane fermentation the whole roof construction may be moved to the side desired along guide rails enabling easy access to the biomass container. In an alternative solution the roof construction may be tipped sideways with the use of connecting hinges, or lifted.

After positioning the gable walls it may be easily sealed at the points of contact with the foundation walls with the aid of siphon or other sealing agent. Application of biogas store construction ensures a circulation of biogas through the gas slots and progressive expulsion of air at the beginning of the process and then the mixture of gases with lower methane value, which enables obtaining biogas with a high fuel value. Application of internal channel to the pulp executed with a perforated barrier enables increasing moisturisation of lower layer of biomass and intensification of the methane fermentation process, with the use of pulp containing biomass and other organic wastes, especially occurring with livestock production. Besides perforations in pulp channel barriers, the channel may be equipped with additional pipes percolating out liquid to biomass. This channel may be easily dismantled after completing the fermentation process. The biogas plant construction and part of the internal installations and post-fermentation deposits remaining in the side zones of the container may be used for plant production. In this case the roof may serve as thermal insulation, and the aeration installations on the base of the container enables aeration post-fermentation deposits and heating them as a result of the action of aerobes . Warm post-fermentation deposits may be used as a substrate in plant production under foil with a simultaneous conversion of post-fermentation deposits into compost.

It is useful if the biogas plant is composed of a group of several or more objects. The construction of the biogas unit enables easy adaptation to fulfil various functions. Apart from plant production on aerated post-fermentation deposits, after the complete clearance of post-fermentation deposits the biogas unit may be used as a dryer using heat from the cogeneration unit or from the fermentation chamber by placing a coil there, before commencing fermentation it may also be used as a biogas storage unit, and the water-tight trough of the biogas unit may be used as a lagoon with a breeding algae with the of use of organic compounds and minerals contained in seepage waters and carbon dioxide and also heat originating from the methane fermentation process, aerobic fermentation or the cogeneration unit. The installations for the production and utilisation of biogas enables the running of a continuous process of methane fermentation in the fermentation chamber with the use of dry and pulp biomass, with the possibility of regulating the process intensity. The channel in the biomass zone for the acceptance and collection of pulp is located between the side zones of the container, in the best place for the development of thermophilic bacteria, for which the biomass collected in the fermentation chamber constitutes heat insulation. The use of the biogas store with gas slots at the level of approx. ¼ of the height of the store and the extraction of carbon dioxide near the roof construction base enables obtaining biogas with enhanced methane value due to the possibility of displacing heavier gas components from the biogas store with return to the fermentation chamber, and gases of a greater density - principally carbon dioxide - to the exterior of the store. The applied heat and mass exchanger enables the degasification and heating seepage water with the use of the heat energy of exhaust is created in the cogeneration system and also its renewed introduction to the fermentation chamber through the biomass moistening installations.

Preferably, if the biogas plant is in regions with a cold climate that the biogas roof is equipped with a chemical de-icing system with sprinkler nozzles placed over the roof ridge.

The invention is shown visually, wherein:
- fig. 1: presents the biogas generating plant with tunnel fermentation chamber and internal installations to produce and store biogas in diagrammatically transverse cross-section,
- fig. 2: presents the heat and mass exchanger diagram, in transverse cross-section,
- fig. 3: presents the biogas generating plant diagram from fig. 1 with installation for producing and utilising biogas together with cogeneration system, heat and mass exchanger and the manure container,
- fig. 4: presents variations of the biogas flow through the biogas store,
- fig. 5: presents details of foundation wall construction in transverse cross-section, e.g. performance,
- fig. 6: presents operational control of biogas generating plant diagrammatically e.g. performance, while,
- fig. 7: presents operational control of plant production process, e.g. performance.

On the drawing the following designations are used: 1 - foundation walls, 1 a - upper biomass zone, 1b - intermediate biomass zone, 1c - lower biomass level, 1d - empty space, 2 - internal pulp channel, 3 - arch roof covering load-bearing construction, 3a - upper foil, 3b - lower foil, 3c - biogas store in, 4 - perforated barrier, 5 - lower ridge of roof covering, 6 - internal pipeline, 7 - moisturising pipeline, 7a - moisturising nozzle, 8 - duct fan, 9 - gas valve, 10 - perforated pipeline, 11 - aeration pipeline, 11a - perforated nozzle, 12 - auger conveyor, 13 - reverse ventilator, 14 - housing, 15 - permanent mass, 16 - absorption charge, 17 - basin, 18 - return pump, 19 - exhaust conduit, 19a - exhaust outlet, 20 - seepage/drainage water pipe, 20a - sprinkler equipment, 21 - exhaust outlet channel, 22 - reverse pipeline, 23 - release valve, 24 - seepage water pump, 25 - heater conduit, 26 - lower slot grating with lower slots, 27 - upper slot grating with side slots, 28 - first water pump, 28a - second water pump, 29 - gas slots, 30 - gas pipeline, 31 - external gas container, 32 - coil, 33 - pipeline, 33a - biogas inlet, 33b - biogas outlet, 34 - pipeline, 35 - valve, 40 - panel, 41 - T-bar, 42 - braces, 43 - insulation material, 44 - longitudinal braces, 44a - runner, 45 - sealing, 46 - keder rail, 47 - securing cord, 48 - channel, 49 - de-icer installations, 50 - sprinkle nozzle.

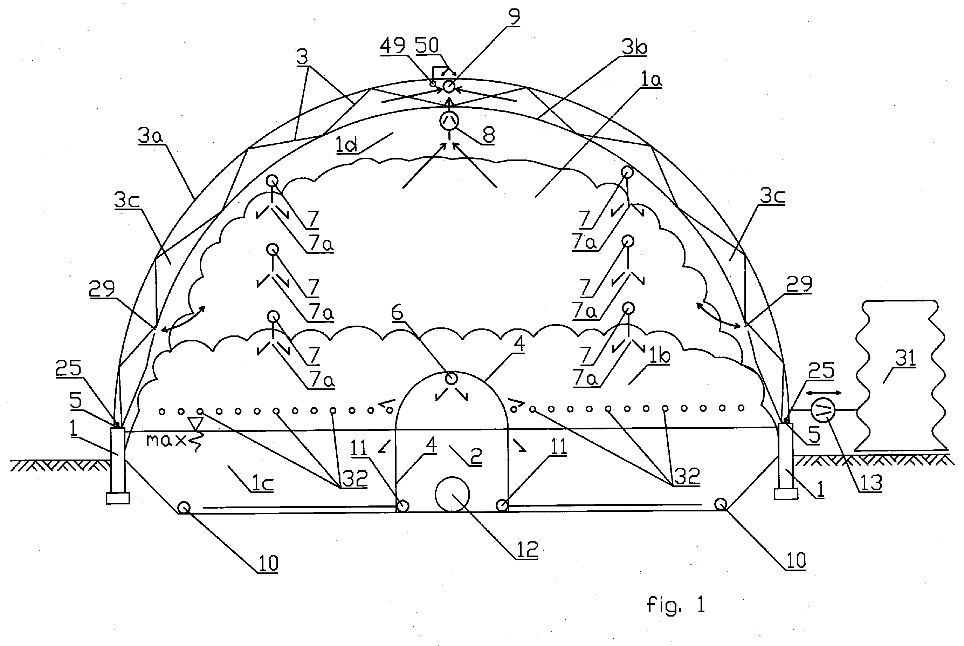

As shown in fig. 1, the biogas generating plant is equipped with biomass container with thermally insulated base and essentially vertical foundation walls 1, installations moisturising biomass with process water, and seepage water recirculation installations, arch roof covering spread on arch load-bearing construction 3 and supported on vertical walls 1 biomass container, gas-tight gable walls, and biogas store 3c formed by double layer of roof covering foil with inlet to biogas store 3c situated at the upper part of tunnel fermentation chamber. The biomass container when seen from above has a rectangular shape. Two layers of foil, upper 3a and lower 3b, forming the biogas store 3c, are secured on opposite sides of the rigid arch load-bearing construction 3 of the roof covering. The load-bearing construction 3 is made of light construction framework. Upper foil 3a is secured to the upper strip, and lower foil 3b is secured to lower strip of load-bearing construction 3. The space between the foils 3a and 3b constitutes the biogas store 3c. In the space between foils 3a and 3b may be placed a gas permeable non-water absorbent thermal insullations layer. The storage space is divided with the aid of foil sleeves protecting the framework construction into separated chambers. In other words each of the arch lattice construction girders 3 is wrapped with a foil sleeve, with at least two openings at the ends of the girder, enabling control of the atmosphere in the sleeve - e.g. by pumping in dry air or gas (for example SO₂ protecting wood against pests). In particular chambers of the biogas store 3c labyrinth barriers or membranes may be mounted facilitating separation of methane and heavy gas articles contained in biogas. Lower foil 3b at the level of approx. 1/4 of the height of the arch load-bearing construction 3 has gas slots 29, and the inlet channel is connected to duct fan 8, forcing the circulation of biogas between biogas store 3c and fermentation chamber. In the lower part of the biogas store 3c is placed a heating conduit 25, for the release of methane from methane hydrate that may form in unfavourable conditions. duct fan 8 is equipped with a flap preventing return flow of gases. At the ventilator suction channel joint 8 is placed a methane value sensor.

The lower edges 5 of the roof covering are supported by appropriately shaped guide runners mounted on upper part of foundation walls of biomass container 1. Appropriately shaped guide runners are e.g. channel iron 48 on the top of foundation walls 1 and channel iron on lower edge 5 of roof covering, with matching measurements so that they may slide together - example the channel iron on lower edge 5 of roof covering covers and embraces channel iron 48 on the top of the foundation wall 1. Roof covering foil is secured with the aid of airtight connections with foundation walls. Furthermore lower edges 5 of roof covering are seated in slide, sliding or rolling, which facilitates lengthways placing of arch roof construction. Alternatively the roof construction may be tipped sideways using hinge connections, or raised. After mounting the gable walls the interior of the biogas generating plant forms a closed tunnel fermentation chamber.

The biomass moisturising installations carrying process seepage liquid to the inside of the biomass is composed of moisturising pipelines 7 with branches to which are attached moisturising nozzles 7a, placed at least on one layer between the biomass container and the upper part of the fermentation chamber. All the moisturising nozzles 7a are placed in the biomass-loading zone at varying heights, depending on the height of the biomass-loading zone, especially in the intermediate area 1b, above the level indicated by the upper edge of the foundation walls 1 of the biomass container. Moisturising nozzles 7a are placed at least in the intermediate zone 1b or at several levels in the interior of biomass, in intermediate zone 1 b and in upper zone 1 a.

In the biomass loading zone there is an internal channel 2 for pulp, preferably to be but not exclusively placed parallel to vertical foundation walls 1, preferably to be separated by the perforated barrier 4 supported by the base of the biomass container and shaped in a reversed letter U. Alternatively channel 2 may be formed as a perforated pipe, suspended above, supported or resting on the base of the biomass container or on the biomass. Perforated sides of the barrier 4 (or - appropriately - perforated pipe) separate channel 2 from biomass container.

In the upper part of the channel 2 is placed the internal pipeline 6 conveying pulp from the pulp container. Perforated barrier 4 enables seepage of excess filtered liquid from channel 2 to side zones of biomass container. In initial phase of the process channel 2 remains empty. After starting the process the pulp is supplemented by the internal pipeline 6. After accepting pulp and drained seepage water, bottom part of the internal channel 2 serves as a sediment trap for solid organic particles in the pulp. To remove compacted post-fermentation deposits from the internal channel 2 to the exterior an auger conveyor is used 12, mounted in the lower part of the channel at the biomass container base. In biomass container side zones, separated by internal channel 2, are placed perforated pipelines 10 draining away seepage water.

The biomass container is equipped with aeration installations with perforated nozzles 11a mounted in side zones by the biomass container base, supplied by aeration pipeline 11. Biomass container seen from above has a rectangular shape, while internal channel 2 may be - for example but is not necessarily - placed in the central part of the container, e.g. parallel with its longest sides. The base of the biomass container is thermally insulated from the substrate. Depending on local conditions that base may be entirely or partially sunk into the ground.

While loading biomass it is laid in the biomass container so that the central part of the fermentation chamber would be filled as much as possible, leaving little free space 1 d between the upper layer of the biomass 1 a and lower foil 3b. After loading, the biomass fermentation chamber is sealed along the base sides and gas-tight closure of gable walls.

Methane fermentation commences following moisturising the biomass with the help of pulp and/or process liquid. Moisturising dry biomass takes place in the lower part of its volume 1 c, wherein during the process of producing biogas, the biomass is in its driest state, a wet state and is immersed in liquid, due to which the intensity of biogas production is regulated by change in the quantity of process liquid supplied by moisturising pipeline 7 or the quantity of pulp supplied through the internal pipeline 6. The process liquid is piped to moisturising nozzles 7a and moisturise is the intermediate layer 1b and lower layer 1c of biomass. Seepage of liquid through perforated barrier 4 moisturise is the lower layer 1 c. Excess liquid in lower layer 1 c is drained away from the biomass container through perforated pipeline 10. The coil 32 placed in the biomass layer serves as a drain for excess heat from fermentation chamber or if necessary may be used to heat deposit in order to stimulate fermentation.

Biogas is formed in the process of methane fermentation and is collected in the upper part of the fermentation chamber then is pumped by duct fan 8 to biogas store 3c after biogas reaches the appropriate methane content value. During the filling of the biogas store the mixture of gases with a low methane value, principally carbon dioxide are extracted from the biogas store 3c through gas slots 29 in lower foil 3b to the fermentation chamber. Along the lower edge of the biogas store are placed electric heating conduits 25, enabling release of methane from methane hydrate. During the circulation of the mixture of gases through the biogas store 3c and gas slots 29 the concentration of methane increases in the upper zone of the biogas store 3c, from where the biogas with enhanced methane value is taken to the cogeneration system through gas valve 9. During collection of biogas through gas valve 9, as a result of differing pressures, a return flow occurs of the mixture of gases from the fermentation chamber through gas slots 29 to the biogas store 3c, which enables equalisation of pressures and increase in the productivity of biogas taken from the store.

Preferably, in case of location of biogas generating plant in regions with a cold climate that the roof of the biogas generating plant is equipped with a chemical de-icing system 49 with sprinkler nozzles 50 mounted above the roof ridge fig. 1.

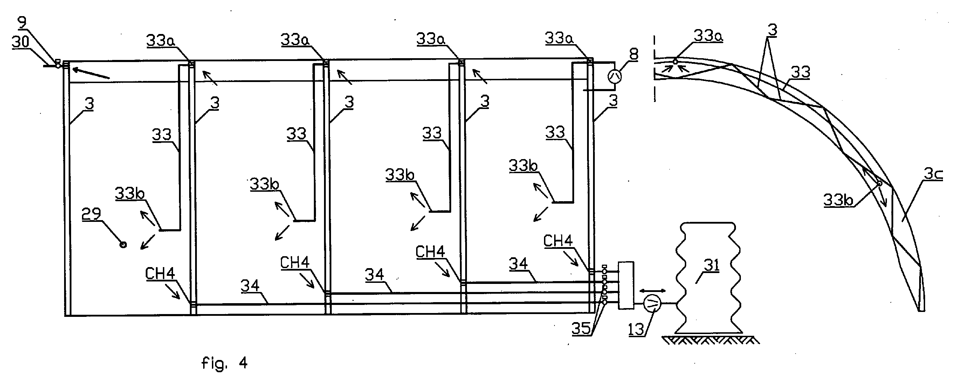

In fig. 4 is shown another variant of the through-flow of biogas through the biogas store. In this variation the ventilator 8 forces the flow of biogas in succession (serial connection) through all the chambers formed in the biogas store. Slot 29 is open in the final chamber and enables return of part of biogas to the fermentation chamber. Through-flow of biogas to successive chambers takes place with the aid of pipelines 33 in such a way that the outlet opening 33a from the chamber is located at the top of the chamber, and inlet 33b to the next chamber - is at a level of approx. 1/2 the height of the store. In both variants the flow of biogas through the store chamber (i.e. through-flow variant parallel and successive) carbon dioxide and other gases of a density greater than methane are taken out of particular storage chambers with the aid of pipelines 34, installed at the bottom of chambers and equipped with valves 35 operated by methane sensors. At the moment of appearance of methane at the outlet to the pipeline the valve should close.

In one of the examples of the implementation of the invention, the upper foil 3a may be tied to a rod, tape or rope 44 placed along the foundation wall 1 with the aid of keder rail 46 and rope 47, with the formation of so-called pockets. 'Keder' fabric is a type of connection well known among specialists. Seal 45 between foil 3a and wall 1 is not in danger from stress. It creates a sealed joint that is simultaneously airtight and elastic, which gives elasticity to the whole construction of the roof covering of the biogas generating plant. This is important and prolongs the duration of the construction exposed to violent or gusty wind action.

It is particularly preferable to use a group of biogas invention objects simultaneously, with appropriate phase staging, known by a biogas generating plant specialist. This would mean - for example - that when the first biogas generating plant would be subject to loading, then the second would be in the process of biomass fermentation and gas collection, while in the third biogas generating plant the process of biomass fermentation is finished and it is ready for loading. Analogously one may use a biogas generating plant arranged in several segments, for which the above-described transfer of phases applies to these segments. The biogas generating plant construction enables easy adaptation for the fulfilment of various functions. Apart from plant production on the aerated post fermentation deposit, after total removal of post fermentation deposits the tunnel may be used as a dryer using heat from the cogeneration system or from the fermentation chamber through the coil placed there, before the beginning of fermentation it may be used as a biogas store, the biogas trough may be used as a lagoon for the breeding of algae using organic compounds and minerals contained in seepage water and carbon dioxide and heat originating from the methane fermentation process, aerobic fermentation or cogeneration unit.

Installations for the production and utilisation of biogas include a tunnel fermentation chamber constituting an internal space within the biogas generating plant space with a convex roof spread over an arch load-bearing construction and biogas store 3c formed by two layers of roof covering foil, installations for moisturising biomass with process liquid, installations recirculation of seepage water, cogeneration system powered by biogas supplied through gas pipeline 30 from biogas store 3c, non-diaphragm heat and mass exchanger with deposit of a large heat capacity and automation system operation of the biogas production and utilisation process.

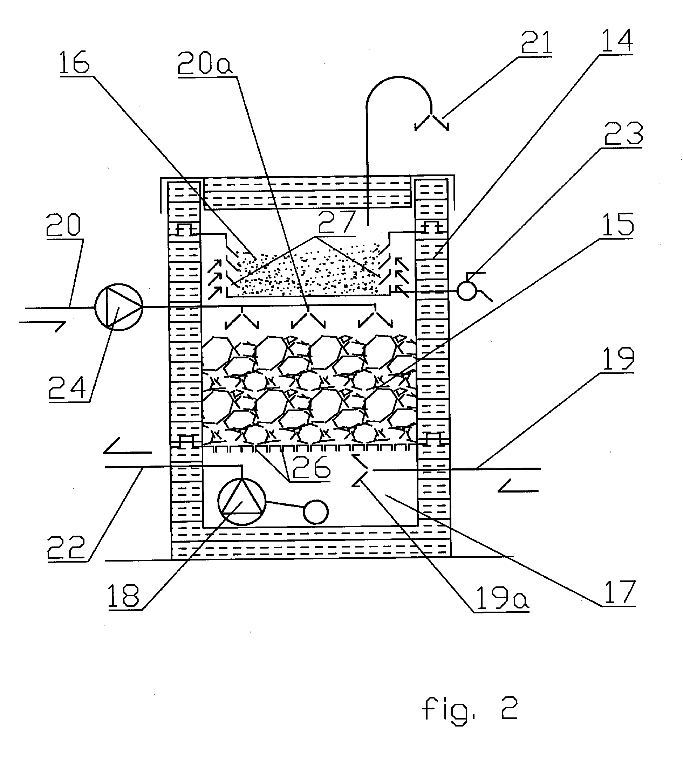

As shown in fig. 2, the heat exchanger is composed of a heat exchanger housing which can be dismantled 14, thermal insulation and permanent mass deposit 15, filled with material of great heat capacity, serving as heat and mass exchanger between seepage water collected by pipeline 10 from the bottom of the container and exhausts produced in the KOG cogeneration unit. Over the permanent mass 15 is mounted sprinkler equipment 20a. Over the sprinkler equipment 20a is the upper gap support grid framework with side slots 27. This framework is filled with absorption charge 16 and absorbing sulphur compounds, especially particles of bog iron ore or steel wool. The permanent mass 15 is placed on the lower gap grid framework with lower gaps 26. Under the permanent mass 15 is placed the basin 17. Between the base 17 and the permanent mass 15 is the exhaust outlet 19a with exhaust conduit 19 taking exhaust from the cogeneration system.

Seepage water carried by sprinkler appliance 20a is passed through the mass in the opposite direction to the exhaust brought to the mass by exhaust conduit 19 from the cogeneration system. Heated water taken from basin 17 is brought from the exchanger through the return pump 18 and pipeline 22 . The return pump 18 is fitted with a liquid level sensor, in particular a floating switch. The exhaust originating through the permanent mass 15 in contact with mass and water gives off heat energy. Heating seepage water causes release of contained gases, principally carbon dioxide and hydrogen sulphide. Gases released from seepage water and exhaust passed through upper slot framework 27 with absorption charge 16, and then expelled to the atmosphere through exhaust outlet channel 21. Condensation collected on the base of the upper slot framework 27 is removed to the exterior by exhaust release 23. The exchanger has a removable housing 14 to enable access to the internal exchanger elements and change of mass 15.

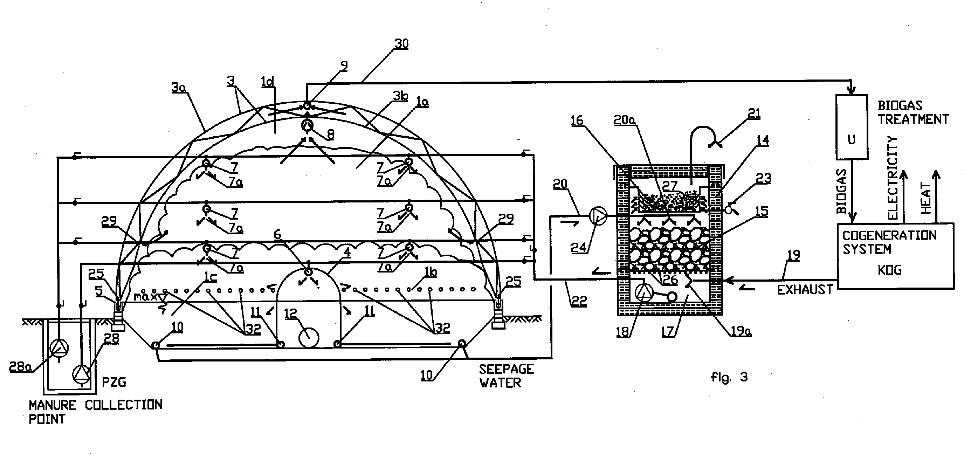

As shown in fig. 3, the biogas generating plant from fig. 1 is connected by gas pipeline 30 with the cogeneration system performing the task of supplying the farm with heat and electricity. The cogeneration system is composed of treated biogas U and cogeneration system KOG, where combustion of biogas drawn from the biogas store 3c takes place. The energy of the exhaust gases occurring as a result of the combustion of biogas is used for heating and to change the chemical composition of seepage water recirculated to the biogas generating plant with the aid of a non-diaphragm heat and mass exchanger of fig. 2. Both foil layers, upper 3a and lower 3b, forming the biogas store 3c, are secured on opposite sides of rigid arch load-bearing construction roof covering 3, wherein the lower foil 3b is at a level of approx. 1/4 of the height of the arch load-bearing construction 3 gas slots 29, and duct fan 8 forcing circulation of biogas from the fermentation chamber to the biogas store 3c is equipped with a flap preventing return flow of gases. Gas slots 29 enable recirculation of gases between the biogas store 3c and the fermentation chamber. Additionally to the lower part of all biogas store chambers 3c is connected by equalising conduit to an external carbon dioxide container 31. A reverse ventilator 13 may be connected to the equalising conduit forcing the flow of gases. External gas container 31 is used for collection of carbon dioxide accumulating in the lower part of the biogas store and filling biogas store volume 3c while taking biogas through gas valve 9. Reverse ventilator 13 assists the exchange of gases.

The installation moisturising biomass with process liquid consists of moisturising pipeline 7 with moisturising nozzles 7a placed in biomass loading zone on varying heights, depending on the height of the biomass loading zone, particularly in intermediate zone 1b. Arrangement of moisturising pipelines 7 with cut off valves of several levels enables moisturising biomass at chosen level. Moisturising of biomass at ever-higher layers increases the speed of biogas production with regard to the moistening of a greater volume of biomass. Depending on requirements to a certain extent one may control the speed of biogas production with the aid of quantities of liquid supplied to space indirectly 1b and by internal channel 2 also with choice of level, at which the liquid will be supplied to the biomass bed. In the case of supplying a greater amount of process liquid, a greater volume of biomass shall be subject to the biochemical process, and thus the output of the biogas production process shall increase. During anaerobic digestion substances are produced that slow down this process, for example through acidifying the mass of biomass. In order to prevent this, the biomass may be initially mixed with chemical substances, correcting the pH of the mass and releasing for example dolomite during moisturising of succeeding layers of biomass.

In the biomass loading zone is channel 2 for pulp, preferably but not exclusively positioned parallel to the essentially vertical wall foundations 1, it may be separated by perforated barrier 4 supported on the biomass container base, preferably a reversed letter shape U. Alternatively the channel 2 may be formed by the perforated pipe, suspended, supported or lying freely on the base of the biomass container or on the biomass. Pulp is conveyed by the internal pipeline 6 situated in the upper part of channel 2. For the removal of solidified post-fermentation deposits from channel 2 to the exterior an auger conveyor 12 is used, mounted in the lower part of channel 2. Perforated barrier 4 (or - appropriately - perforated pipe) enables permeation of excess cleaned fluid from channel 2 to side zones of the biomass container. Another solution for draining seepage is the application of additional moisturising pipes connected with channel 2. In the side zones of the biomass container, divided by the internal channel 2, perforated pipelines 10 are placed that drain away seepage water through seepage water pipeline 20 to heat and mass exchanger. From the heat and mass exchanger the heated seepage water returns by return pipeline 22 to moisturising pipelines 7 installations moisturising biomass with process liquid and also depending on requirements through the internal pipeline 6 to channel 2. Excess seepage water may also be taken to manure collection point PZG or to the outside of the installations.

Process liquid necessary to start and maintain methane fermentation may be periodically collected together with pulp from manure collection point PZG with the aid of first water pump 28 and pushed into channel 2 with the internal pipeline 6. Equally process liquid with small amounts of solid particles may be taken from manure collection point PZG by second water pump 28a, situated in a higher layer of manure heap, from where it is taken to moisturising pipelines 7. In another solution pump 28, depending on requirements, may be placed at the appropriate level with the aid of guide rails.

In relation to the process of fermentation volume of substrates in the intermediate zone 1 b and lower 1 c shall shrink. The biomass volume in these zones will be supplemented by gravitational sinking of dry biomass stored in upper zone 1a. Dry biomass in upper zone 1a fulfils the function in the biogas production process of heat insulation, storage of substrates, transitional storage of biogas and separator of gases. In step with the duration of the fermentation process upper zone 1a shall be reduced. Completion of the fermentation process takes place as a result of the pumping out of liquid from the biomass container and the pumping in to the biomass mass of compressed air through aeration pipeline 11. The development of aerobic bacteria causes an increase of the substrate temperature. The substrate temperature may be regulated by the amount of air pumped into perforated nozzles 11a supplied by aeration pipeline 11 and by heat extraction with the aid of coil 32. The period of transformation of post fermentation deposits into compost may be used for agricultural production under foil on heated substrate consisting primarily of post-fermentation deposits.

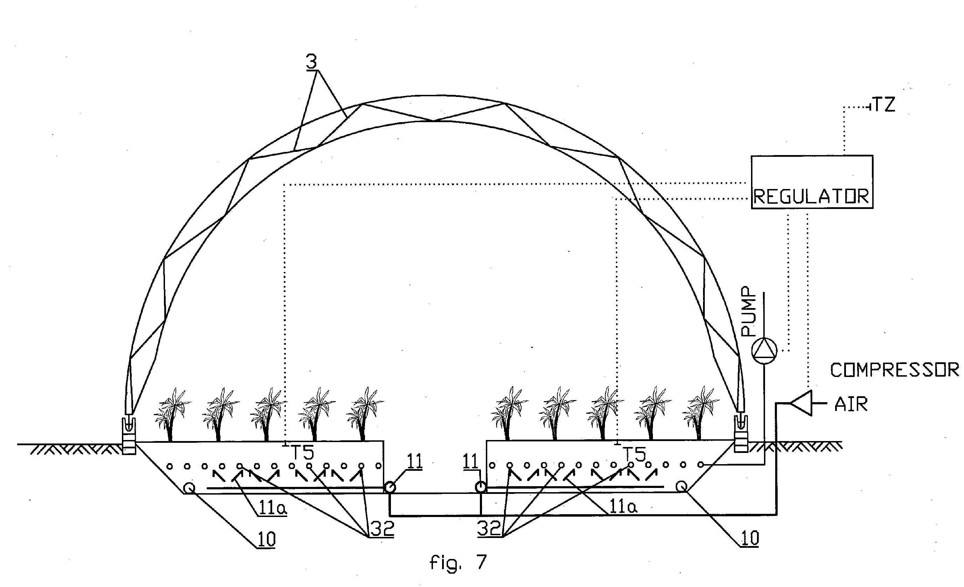

Adaptation of the biogas generating plant to cultivation of plants depends on removal of post fermentation deposits from channel 2, removal of excess layer of deposits in side zones of biomass container, dismantling upper part of channel 2, connection of a compressor to pipeline 11, pumping out excess water and possibly laying upper layer of cultivation soil appropriate for the cultivated plants. Compressed air supplied through pipeline 11 initiates and supports aerobic fermentation in residue deposits (fig. 7). Control of the processes described in the means of production of biogas and production of plants is by manual starting and stopping of pumps, compressors, opening and closing appropriate valves and connecting and disconnecting biogas container.

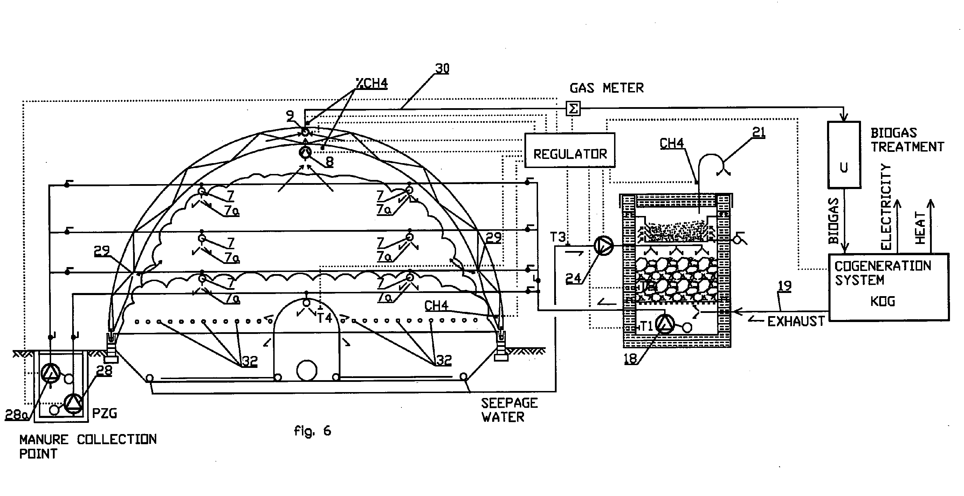

According to fig. 6 and 7, the construction of the biogas generating plant and installations according to the invention enables automatic control of production processes, storage and burning of biogas in KOG cogeneration system with the assumption that in subsequent units of time, as far as possible production of approximately the same volumes of biogas shall be produced. Starting or disconnection of water pumps28 and 28a take place on the basis of algorithm comparing quantities of biogas produced in successive time units. Water pumps 28 and 28a are protected against running without water by float switches. The volume of biogas shall be measured on the basis of basic data from GAS METER and all by the time of work and productivity of duct fan 8. Connection of duct fan 8 takes place after achieving methane concentration in biogas that is programmed in regulator and appropriate to the correct operation of recipient. In the control system the methane concentration measure is set with the aid of %CH4 sensors placed in the upper part of the fermentation chamber, by the suction socket of duct fan 8 and gas pipeline 30. Disconnection of duct fan 8 takes place after reduction of methane percentage value in biogas below the required value at the suction socket of the ventilator. Taking of biogas to the cogeneration system occurs after opening gas valve 9. Gas valve 9 is closed if the methane percentage value in biogas in gas pipeline 30 falls below the required value. Removal of carbon dioxide accumulated in lower parts of particular biogas store chambers 3c will be performed on the condition that sensors CH4 placed in chambers will not indicate the presence of methane. In such a case valve 35 remains open and ventilator 13 pumps carbon dioxide to store 31. (fig 4.) Operation of heat and mass exchanger is controlled with the aid of temperature sensors T1, T2 and T3 and also methane sensor CH4 mounted in exhaust pipe conduit 21. Connection of seepage water pump 24 takes place after reaching specified temperature of permanent charge 15 (sensor T2), and its disconnection after reaching specified temperature of water in basin 17 (sensor T1). Disconnection of seepage water pump 24 may take place after discovery of presence of methane in exhaust pipe channel 21 (sensor CH4). Other temperature sensors shall measure temperatures of biochemical processes in shares in biogas generating plant points and attached installations. This also applies to monitoring of substrate temperature in plant production phase (sensor T5) and operation of a compressor and pump causing circulation through coil 32. Operating and control program should have regard for time delay of substrate reaction to quantity of air supplied to substrate and correlate work of compressor on the basis of forecast and actual weather conditions (sensor TZ).

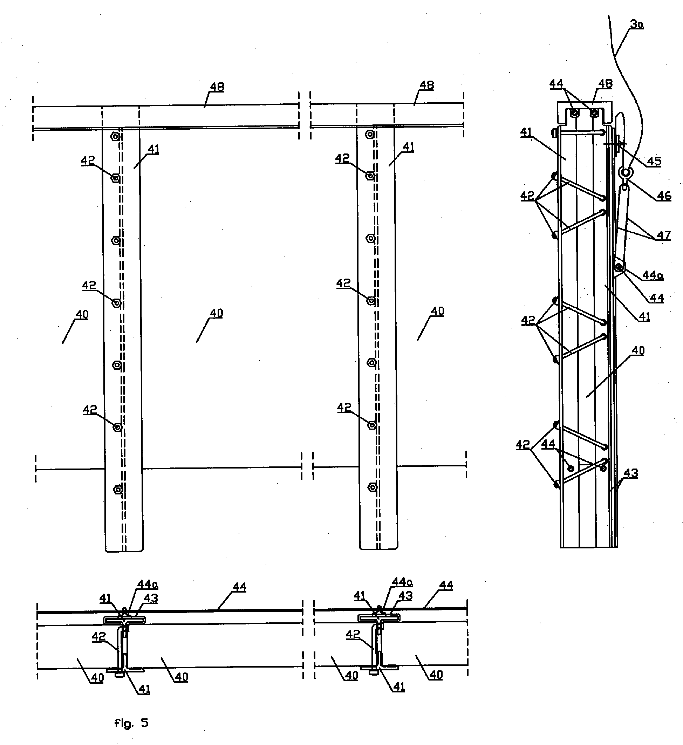

Foundation walls 1 shown in fig. 3, 6 and 7 are built with brickwork or concrete. Alternative constructions of foundation walls are shown in fig. 1 and 5. In this case foundations 1 include panels 40, mounted between posts. As shown in fig 5, foundation wall posts are composed of two T-bars 41 drawings with braces 42. Between T-bars 41 are foundation wall elements with high thermal insulation. External T-bar 41 is wrapped in insulation material 43. Such post-construction limits loss of heat to the exterior of foundation walls of biogas generating plant.

## Claims

1. Biogas generating plant with tunnel fermentation chamber, equipped with biomass container with thermally insulated base and essentially vertical foundation walls, installations for moisturising biomass with a process liquid, installations for recirculation of seepage water, arch roof covering spread over arch load-bearing construction and supported on essentially vertical biomass container walls, gas-tight gable walls and biogas store formed by double layer of roof covering foil with inlet to biogas store situated in upper part of fermentation chamber, **characterised in that** two layers of foil, upper (3a) and lower (3b), forming the biogas store (3c), are secured on opposite sides of the rigid arch roof covering load-bearing construction, the lower foil (3b) has gas slots (29) at approx. 1/4 of the height of the arch load-bearing construction, and a duct fan (8) forcing circulation of biogas to biogas store (3c) is connected into the inlet channel, wherein lower edges (5) of roof covering are supported on upper part of essentially vertical foundation walls (1) of the biomass container, while the installations for moisturising biomass with process liquid have moisturising nozzles (7a), placed in the biomass loading zone and variable heights, depending on the height of the biomass loading zone, preferably in intermediate zone (1b), and furthermore in the biomass loading zone there is a channel (2) for pulp, preferably created by a perforated barrier (4) supported on the base of biomass container or constituting a perforated pipe, suspended, supported or resting freely in a biomass layer.

2. Biogas generating plant according to claim 1, **characterised in that** the lower edges (5) of the roof covering are slideably mounted and form a gas tight connection on the appropriately shaped upper part of the vertical foundation walls (1), specifically with the aid of slides or guide rails comprising channel irons, or the lower edges (5) of the roof covering are slideably mounted in troughs executed in the upper part of vertical foundation walls (1), which troughs form gas tight connections of the lower edges (5), especially siphon connections.

3. Biogas generating plant according to claim 1 or 2, **characterised in that** the moisturising nozzles (7a) are located in the biomass loading zone on one level or at various levels between the biomass container and the upper part of the fermentation chamber.

4. Biogas generating plant according to claim 1, 2 or 3, **characterised in that** in the upper part of the channel (2) for pulp there is placed an internal pipeline (6) conveying pulp, and in the lower part of the channel (2) for pulp there is installed an auger conveyor (12) to convey compacted pulp to the exterior.

5. Biogas generating plant according to any of the preceding claims from 1 to 4, **characterised in that** the biomass container is equipped with aeration installations with perforated nozzles (11a) mounted in side zones at the biomass container base, supplied by aeration pipeline (11).

6. Biogas generating plant according to any of the preceding claims from 1 to 5, **characterised in that** it is equipped with a coil (32) placed in the biomass layer, to remove surplus heat from the fermentation chamber or to heat mass.

7. Biogas generating plant according to any of the preceding claims from 1 to 6, **characterised in that** the rigid arch load-bearing construction (3) of the roof covering is placed in a gas-tight foil sleeve, insulating the construction (3) from the gaseous atmosphere in the biogas store.

8. Biogas generating plant according to claim 7, **characterised in that** said foil sleeve has inspection openings for inlet of appropriate gases protecting the construction against deterioration and for controlling moisture of the atmosphere inside the sleeve.

9. Biogas generating plant according to any of the preceding claims from 1 to 8, **characterised in that** the essentially vertical foundation walls (1) are made of a thermoinsulating material, preferably using brickwork or made of thermoinsulating panels (40), placed between pillars.

10. Installation to produce and utilise biogas, comprising a tunnel fermentation chamber, constituting a space inside a biogas generator with a convex roof covering spread over an arch load-bearing structure, a gas store formed by two layers of roof covering foil with an inlet to the gas store situated in the upper part of the fermentation chamber, installations for moisturising biomass with a process liquid, installations for recirculation of seepage water, a cogeneration system powered by biogas supplied through a gas pipeline from the biogas store and an automated system for controlling the process of production and utilisation of biogas, **characterised in that** two layers of foil, upper (3a) and lower (3b), forming the biogas store (3c), are secured on opposite sides of the rigid arch roof covering load-bearing construction, the lower foil (3b) has gas slots (29) at approx. 1/4 of the height of the arch load-bearing construction, and a duct fan (8) forcing circulation of biogas to biogas store (3c) is connected into the inlet channel, wherein lower edges (5) of roof covering are supported on upper part of essentially vertical foundation walls (1) of the biomass container, while the installations for moisturising biomass with process liquid have moisturising nozzles (7a), placed in the biomass loading zone and variable heights, depending on the height of the biomass loading zone, preferably in intermediate zone (1b), and furthermore in the biomass loading zone there is a channel (2) for pulp, preferably created by a perforated barrier (4) supported on the base of biomass container or constituting a perforated pipe, suspended, supported or resting freely in a biomass layer, supplied with pulp through an internal pipeline (6) situated in the channel (2) for pulp, wherein the installations for recirculation of seepage water include a non-membrane heat and mass exchanger connected through a seepage water pipeline (20) with the biomass container and a return pipeline (22) with installations for moisturising biomass with a process liquid, and wherein the non-diaphragm heat and mass exchanger has a permanent mass (15) of a high heat capacity, through which seepage water is passed in the opposite direction to exhaust gases supplied to the permanent mass by an exhaust conduit (19) from the cogeneration system.

11. Installation according to claim 10, **characterised in that** the seepage water pipeline (20) is connected with a perforated pipeline (10) in the biomass container and with a seepage water pump (24) connected on compression side with a moisturising appliance (20a) placed above the permanent mass (15) of a non-diaphragm heat and mass exchanger, while under the permanent mass (15) there is placed a basin (17) to receive water released from the biomass, wherein the water from basin (17) is pushed to the return pipeline (22) by a return pump (18).

12. Installation according to claim 10 or 11, **characterised in that** the moisturising nozzles (7a) are situated in the biomass loading zone at one level or at various levels between the biomass container and the upper part of the fermentation chamber.

13. Installation according to claim 10, 11 or 12, **characterised in that** in the upper part of the channel (2) for pulp there is placed an internal pipeline (6) conveying pulp, and in the lower part of the channel (2) for pulp there is installed an auger conveyor (12) to convey compacted pulp to the exterior.

14. Installation according to any of the preceding claims from 10 to 13, **characterised in that** the biomass container is equipped with aeration installations with perforated nozzles (11a) mounted in side zones at the biomass container base, supplied by aeration pipeline (11).

15. Installation according to any of the preceding claims from 10 to 14, **characterised in that** an external gas container (31) for carbon dioxide is connected by an equalising conduit to the lower part of the biogas store (3c), wherein preferably a reverse ventilator (13) for forcing gas through-flow is connected to the equalising conduit.

## Patentansprüche

1. Biogaserzeugungsanlage mit Tunnelgärungskammer, ausgerüstet mit einem Biomassebehälter mit thermisch isolierter Basis und im Wesentlichen vertikalen Fundamentwänden, Einrichtungen zur Befeuchtung der Biomasse mit einer Prozessflüssigkeit, Einrichtungen zur Rückführung des Sickerwassers, gewölbter Dacheindeckung, die sich über einem gewölbten Tragwerk erstreckt und auf im Wesentlichen vertikalen Biomassebehälterwänden gestützt ist, gasdichten Giebelwänden und einem Biogasspeicher, der von einer Doppelschicht der Dacheindeckungsfolie gebildet wird, wobei sich der Einlass zum Biogasspeicher im oberen Teil der Gärungskammer befindet, **dadurch gekennzeichnet, dass** die zwei Folienschichten, die obere (3a) und die untere (3b), die den Biogasspeicher (3c) bilden, auf gegenüberliegenden Seiten des steifen gewölbten Tragwerks der Dacheindeckung fixiert sind, die untere Folie (3b) auf ungefähr 1/4 der Höhe des gewölbten Tragwerks mit Gasschlitzen (29) versehen ist, und ein Führungsgebläse (8), das die Beförderung des Biogases in den Biogasspeicher (3c) erzwingt, an ein Einströmkanal angeschlossen ist, wobei die unteren Ränder (5) der Dacheindeckung auf dem oberen Teil der im Wesentlichen vertikalen Fundamentwände (1) des Biomassebehälters gestützt sind, wobei die Einrichtungen zur Befeuchtung der Biomasse mit Prozessflüssigkeit mit Befeuchtungsdüsen (7a) ausgerüstet sind, die sich im Biomassebeschickungsbereich auf variabler Höhe, in Abhängigkeit von der Höhe des Biomassebeschickungsbereichs, bevorzugt im Zwischenbereich (1b), befinden, und sich im Biomassebeschickungsbereich weiterhin ein Kanal (2) für die Pulpe befindet, der bevorzugt durch eine perforierte, auf der Basis des Biomassebehälters gestützte Absperrung (4) gebildet wird oder ein perforiertes Rohr ist, das in der Biomasseschicht abgehängt oder abgestützt ist oder frei darin liegt.

2. Biogaserzeugungsanlage nach Anspruch 1, **dadurch gekennzeichnet, dass** die unteren Ränder (5) der Dacheindeckung verschiebbar angebracht sind und eine gasdichte Verbindung auf dem entsprechend geformten oberen Teil der vertikalen Fundamentwände (1) bilden, insbesondere mithilfe von Schiebern oder Führungsschienen, umfassend U-Stähle, oder die unteren Ränder (5) der Dacheindeckung in Rinnen verschiebbar angebracht sind, die im oberen Teil der vertikalen Fundamentwände (1) ausgeführt sind und gasdichte Verbindungen der unteren Ränder (5), insbesondere Siphonverbindungen, bilden.

3. Biogaserzeugungsanlage nach Anspruch 1 oder 2, **dadurch gekennzeichnet, dass** sich die Befeuchtungsdüsen (7a) im Biomassebeschickungsbereich auf einem Niveau oder auf verschiedenen Niveaus zwischen dem Biomassebehälter und dem oberen Teil der Gärungskammer befinden.

4. Biogaserzeugungsanlage nach einem der Ansprüche 1, 2 oder 3, **dadurch gekennzeichnet, dass** sich im oberen Teil des Pulpen-Kanals (2) eine innere Leitung (6) befindet, die Pulpe befördert, und im unteren Teil des Pulpen-Kanals (2) eine Förderschnecke (12) zur Beförderung der zusammenpressten Pulpe nach außen angebracht ist.

5. Biogaserzeugungsanlage nach einem der vorhergehenden Ansprüche von 1 bis 4, **dadurch gekennzeichnet, dass** der Biomassebehälter mit Belüftungseinrichtungen mit perforierten, in den Seitenbereichen an der Biomassebehälterbasis angebrachten Düsen (11a) ausgerüstet ist, die von einer Belüftungsleitung (11) versorgt werden.

6. Biogaserzeugungsanlage nach einem der vorhergehenden Ansprüche von 1 bis 5, **dadurch gekennzeichnet, dass** sie mit einer in der Biomasseschicht platzierten Wicklung (32) ausgerüstet ist, um überschüssige Wärme aus der Gärungskammer zu entfernen oder die Masse zu erhitzen.

7. Biogaserzeugungsanlage nach einem der vorhergehenden Ansprüche von 1 bis 6, **dadurch gekennzeichnet, dass** sich das steife gewölbte Tragwerk (3) der Dacheindeckung in einem gasdichten Folienschlauch befindet, der das Tragwerk (3) von der Gasatmosphäre im Biogasspeicher isoliert.

8. Biogaserzeugungsanlage nach Anspruch 7, **dadurch gekennzeichnet, dass** der genannte Folienschlauch Beobachtungsöffnungen zum Einlassen von entsprechenden Gasen, die die Struktur vor dem Verfall schützen, und zur Überprüfung der Feuchtigkeit der Atmosphäre innerhalb des Schlauchs aufweist.

9. Biogaserzeugungsanlage nach einem der vorhergehenden Ansprüche von 1 bis 8, **dadurch gekennzeichnet, dass** die im Wesentlichen vertikalen Fundamentwände (1) aus einem thermoisolierenden Material, bevorzugt unter Verwendung von Maurerarbeit, oder aus thermoisolierenden Platten (40) hergestellt sind, die zwischen Pfeilern angeordnet sind.

10. Einrichtung zur Herstellung und Verwertung von Biogas, umfassend eine Tunnelgärungskammer, die einen Raum innerhalb des Biogasgenerators mit einer gewölbten, sich über einem gewölbten Tragwerk erstreckenden Dacheindeckung bildet, einem Gasspeicher, der von zwei Schichten der Dacheindeckungsfolie mit einem sich im oberen Teil der Gärungskammer befindenden Einlass zum Gasspeicher gebildet wird, Einrichtungen zur Befeuchtung der Biomasse mit einer Prozessflüssigkeit, Einrichtungen zur Rückführung des Sickerwassers, einem mit Biogas, das durch eine Gasleitung aus dem Biogasspeicher zugeführt wird, betriebenen Kraft-Wärme-Kopplungs-System und einem automatisierten System zur Steuerung des Biogas-Herstellungs- und -Verwertungs-Prozesses, **dadurch gekennzeichnet, dass** die zwei Folienschichten, die obere (3a) und die untere (3b), die den Biogasspeicher (3c) bilden, auf gegenüberliegenden Seiten des steifen gewölbten Tragwerks der Dacheindeckung fixiert sind, die untere Folie (3b) auf ungefähr 1/4 der Höhe des gewölbten Tragwerks mit Gasschlitzen (29) versehen ist, und ein Führungsgebläse (8), das die Beförderung des Biogases in den Biogasspeicher (3c) erzwingt, an ein Einströmkanal angeschlossen ist, wobei die unteren Ränder (5) der Dacheindeckung auf dem oberen Teil der im Wesentlichen vertikalen Fundamentwände (1) des Biomassebehälters gestützt sind, wobei die Einrichtungen zur Befeuchtung der Biomasse mit Prozessflüssigkeit mit Befeuchtungsdüsen (7a) ausgerüstet sind, die sich im Biomassebeschickungsbereich auf variabler Höhe, in Abhängigkeit von der Höhe des Biomassebeschickungsbereichs, bevorzugt im Zwischenbereich (1b) befinden, und sich im Biomassebeschickungsbereich weiterhin ein Kanal (2) für die Pulpe befindet, der bevorzugt durch eine perforierte, auf der Basis des Biomassebehälters gestützte Absperrung (4) gebildet wird oder ein perforiertes Rohr ist, das in der Biomasseschicht abgehängt oder abgestützt ist oder frei darin liegt, das durch eine innere, im Pulpen-Kanal (2) liegende Leitung (6) mit Pulpe versorgt wird, wobei die Einrichtungen zur Rückführung des Sickerwassers einen direkten Wärme- und Massetauscher umfassen, der mittels einer Sickerwasserleitung (20) mit dem Biomassebehälter und einer Rückleitung (22) mit Einrichtungen zur Befeuchtung der Biomasse mit Prozessflüssigkeit verbunden ist, und wobei der direkte Wärme- und Massetauscher eine permanente Masse (15) mit einer hohen Wärmekapazität aufweist, durch welche das Sickerwasser in einer den Abgasen - mit denen die permanente Masse durch die Abgasleitung (19) aus dem Kraft-Wärme-Kopplungs-System beaufschlagt wird - entgegengesetzten Richtung geleitet wird.

11. Einrichtung nach Anspruch 10, **dadurch gekennzeichnet, dass** die Sickerwasserleitung (20) mit einer perforierten Leitung (10) im Biomassebehälter und mit einer Sickerwasserpumpe (24) verbunden ist, die auf der Kompressionsseite mit einer Befeuchtungsvorrichtung (20a) verbunden ist, die sich über der permanenten Masse (15) eines direkten Wärme- und Massetauschers befindet, während sich unter der permanenten Masse (15) ein Becken (17) zur Aufnahme des aus der Biomasse freigesetzten Wassers befindet, wobei dass Wasser aus dem Becken (17) von der Rückförderpumpe (18) in die Rückleitung (22) befördert wird.

12. Einrichtung nach Anspruch 10 oder 11, **dadurch gekennzeichnet, dass** sich die Befeuchtungsdüsen (7a) im Biomassebeschickungsbereich auf einem Niveau oder auf verschiedenen Niveaus zwischen dem Biomassebehälter und dem oberen Teil der Gärungskammer befinden.

13. Einrichtung nach einem der Ansprüche 10, 11 oder 12, **dadurch gekennzeichnet, dass** sich im oberen Teil des Pulpen-Kanals (2) eine innere Leitung (6) befindet, die Pulpe befördert, und im unteren Teil des Pulpen-Kanals (2) eine Förderschnecke (12) zur Beförderung der zusammenpressten Pulpe nach außen angebracht ist.

14. Einrichtung nach einem der vorhergehenden Ansprüche von 10 bis 13, **dadurch gekennzeichnet, dass** der Biomassebehälter mit Belüftungseinrichtungen mit perforierten, in den Seitenbereichen an der Biomassebehälterbasis angebrachten Düsen (11a) ausgerüstet ist, die von einer Belüftungsleitung (11) versorgt werden.

15. Einrichtung nach einem der vorhergehenden Ansprüche von 10 bis 14, **dadurch gekennzeichnet, dass** ein externer Gasbehälter (31) für Kohlenstoffdioxid mittels einer Ausgleichsleitung mit dem unteren Teil des Biogasspeichers (3c) verbunden ist, wobei vorzugsweise ein Rücklaufventilator (13) zur Erzwingung eines Gasdurchflusses mit der Ausgleichsleitung verbunden ist.

## Revendications

1. Usine de production de biogaz avec une chambre de fermentation de tunnel, équipée d'un conteneur de biomasse avec une base isolée thermiquement et des parois de fondation essentiellement verticales, des installations pour hydrater la biomasse par un liquide de traitement, des installations pour remettre en circulation l'eau d'infiltration, un revêtement de toit en arc étalé sur une construction porteuse en arc et soutenu sur des parois essentiellement verticales du conteneur de biomasse, des parois de pignon étanches au gaz et un magasin de biogaz formé par double couche de la feuille de recouvrement de toit avec une entrée au magasin de biogaz située dans la partie supérieure de la chambre de fermentation, **caractérisée en ce que** deux couches de feuille, supérieure (3a) et inférieure (3b), formant le magasin de biogaz (3c), sont fixées sur des côtés opposés du toit en arc rigide recouvrant la construction porteuse, la feuille inférieure (3b) présente des fentes de gaz (29) à env. 1/4 de la hauteur de la construction porteuse en arc, et un ventilateur de conduit (8) forçant la circulation du biogaz vers le magasin de biogaz (3c) est connecté dans le canal d'entrée, où des bords inférieurs (5) du revêtement de toit sont supportés sur la partie supérieure des parois de fondation essentiellement verticales (1) du conteneur de biomasse, alors que les installations pour hydrater la biomasse par un liquide de traitement ont des buses hydratantes (7a), placées dans la zone de chargement de la biomasse et à hauteurs variables, en fonction de la hauteur de la zone de chargement de la biomasse, de préférence dans la zone intermédiaire (1 b), et de plus dans la zone de chargement de la biomasse il y a un canal (2) pour la pulpe, de préférence créé par une barrière perforée (4) supportée sur la base du conteneur de biomasse ou constituant un tuyau perforé suspendu, supporté ou reposant librement dans une couche de la biomasse.

2. Usine de production de biogaz selon la revendication 1, **caractérisée en ce que** les bords inférieurs (5) du revêtement de toit sont montés coulissants et forment une connexion étanche au gaz sur la partie supérieure de forme appropriée des parois de fondation verticales (1), particulièrement à l'aide des glissières ou des rails de guidage comprenant des fers linéaires, ou les bords inférieurs (5) du revêtement de toit sont montés coulissants dans des caniveaux réalisés dans la partie supérieure des parois de fondation verticales (1), lesdits caniveaux formant des connexions étanches au gaz des bords inférieurs (5), en particulier des connexions de siphon.

3. Usine de production de biogaz selon la revendication 1 ou 2, **caractérisée en ce que** les buses hydratantes (7a) sont situées dans la zone de chargement de la biomasse à un seul niveau ou aux divers niveaux entre le conteneur de biomasse et la partie supérieure de la chambre de fermentation.

4. Usine de production de biogaz selon la revendication 1, 2 ou 3, **caractérisée en ce que** dans la partie supérieure du canal (2) pour la pulpe, il y a une conduite intérieure (6) pour le transport de la pulpe, et dans la partie inférieure du canal (2) pour la pulpe, il y a un transporteur à vis (12) pour transporter la pulpe compactée vers l'extérieur.

5. Usine de production de biogaz selon l'une quelconque des revendications précédentes de 1 à 4, **caractérisée en ce que** le conteneur de biomasse est équipé d'installations d'aération avec des buses perforées (11a) montées dans des zones latérales à la base du conteneur de biomasse, fournies par une conduite d'aération (11).

6. Usine de production de biogaz selon l'une quelconque des revendications précédentes de 1 à 5, **caractérisée en ce qu'**elle est équipée d'une bobine (32) placée dans la couche de biomasse pour éliminer la chaleur excédentaire de la chambre de fermentation ou pour chauffer la masse.

7. Usine de production de biogaz selon l'une quelconque des revendications précédentes de 1 à 6, **caractérisée en ce que** la construction porteuse rigide en arc (3) du revêtement de toit est placée dans un manchon en feuille étanche au gaz, isolant la construction (3) de l'atmosphère gazeuse dans le magasin de biogaz.

8. Usine de production de biogaz selon la revendication 7, **caractérisée en ce que** ledit manchon en feuille présente des ouvertures d'inspection pour l'entrée des gaz appropriés pour protéger la construction contre la détérioration et pour contrôler l'humidité de l'atmosphère à l'intérieur du manchon.

9. Usine de production de biogaz selon l'une quelconque des revendications précédentes de 1 à 8, **caractérisée en ce que** les parois de fondation essentiellement verticales (1) sont constituées d'un matériau thermo-isolant, de préférence en utilisant maçonnerie ou constituées des panneaux thermo-isolants (40), placés entre des piliers.

10. Installation pour produire et utiliser le biogaz, comprenant une chambre de fermentation de tunnel, constituant un espace à l'intérieur d'un générateur de biogaz avec un revêtement de toit convexe étalé sur une structure porteuse en arc, un magasin de gaz formé par deux couches du revêtement de toit en feuille avec une entrée au magasin de gaz située dans la partie supérieure de la chambre de fermentation, des installations pour hydrater la biomasse par un liquide de traitement, des installations pour remettre en circulation l'eau d'infiltration, un système de cogénération alimenté par le biogaz fourni à travers une conduite de gaz du magasin de biogaz et un système automatisé pour contrôler le processus de production et d'utilisation du biogaz, **caractérisée en ce que** deux couches de feuille, supérieure (3a) et inférieure (3b), formant le magasin de biogaz (3c), sont fixées sur des côtés opposés du toit en arc rigide recouvrant la construction porteuse, la feuille inférieure (3b) présente des fentes de gaz (29) à env. 1/4 de la hauteur de la construction porteuse en arc, et un ventilateur de conduit (8) forçant la circulation du biogaz vers le magasin de biogaz (3c) est connecté dans le canal d'entrée, où des bords inférieurs (5) du revêtement de toit sont supportés sur la partie supérieure des parois de fondation essentiellement verticales (1) du conteneur de biomasse, alors que les installations pour hydrater la biomasse par un liquide de traitement ont des buses hydratantes (7a), placées dans la zone de chargement de la biomasse et à hauteurs variables, en fonction de la hauteur de la zone de chargement de la biomasse, de préférence dans la zone intermédiaire (1 b), et de plus dans la zone de chargement de la biomasse il y a un canal (2) pour la pulpe, de préférence créé par une barrière perforée (4) supportée sur la base du conteneur de biomasse ou constituant un tuyau perforé suspendu, supporté ou reposant librement dans une couche de la biomasse, fourni avec de la pulpe à travers une conduite intérieure (6) située dans le canal (2) pour la pulpe, dans laquelle les installations pour remettre en circulation l'eau d'infiltration comprennent un échangeur de chaleur et de masse sans membrane connecté par une conduite (20) d'eau d'infiltration et une conduite de retour (22) avec les installations pour hydrater la biomasse par un liquide de traitement et dans laquelle l'échangeur de chaleur et de masse sans diaphragme a une masse permanente (15) d'une capacité thermique élevée à travers laquelle l'eau d'infiltration est passée dans la direction opposée aux gaz d'échappement fournis vers la masse permanente par un conduit d'échappement (19) à partir du système de cogénération.

11. Installation selon la revendication 10, **caractérisée en ce que** la conduite d'eau d'infiltration (20) est connectée à une conduite perforée (10) dans le conteneur de biomasse et à une pompe à eau d'infiltration (24) connectée sur le côté de compression à un appareil d'hydratation (20a) placé au-dessus de la masse permanente (15) d'un échangeur de chaleur et de masse sans diaphragme, alors que sous la masse permanente (15) il y a un bassin (17) pour recevoir de l'eau libérée de la biomasse, dans laquelle l'eau du bassin (17) est poussée vers la conduite de retour (22) par une pompe de retour (18).

12. Installation selon la revendication 10 ou 11, **caractérisée en ce que** les buses hydratantes (7a) sont situées dans la zone de chargement de la biomasse à un seul niveau ou aux divers niveaux entre le conteneur de biomasse et la partie supérieure de la chambre de fermentation.

13. Installation selon la revendication 10, 11 ou 12, **caractérisée en ce que** dans la partie supérieure du canal (2) pour la pulpe, il y a une conduite intérieure (6) pour le transport de la pulpe, et dans la partie inférieure du canal (2) pour la pulpe, il y a un transporteur à vis (12) pour transporter la pulpe compactée vers l'extérieur.

14. Installation selon l'une quelconque des revendications précédentes de 10 à 13, **caractérisée en ce que** le conteneur de biomasse est équipé d'installations d'aération avec des buses perforées (11a) montées dans des zones latérales à la base du conteneur de biomasse, fournies par une conduite d'aération (11).

15. Installation selon l'une quelconque des revendications précédentes de 10 à 14, **caractérisée en ce qu'**un conteneur extérieur de gaz (31) pour le dioxyde de carbone est connecté par un conduit d'égalisation à la partie inférieure du magasin de biogaz (3c), dans laquelle de préférence un ventilateur inversé (13) pour forcer le gaz d'écoulement traversant est connecté au conduit d'égalisation.
